(19) **Europäisches Patentamt / European Patent Office / Office européen des brevets**

(11) **EP 1 953 127 A2**

(12) **EUROPÄISCHE PATENTANMELDUNG**

(43) Veröffentlichungstag:
06.08.2008 Patentblatt 2008/32

(51) Int Cl.:
*C07B 61/00* (2006.01)      *C07C 2/66* (2006.01)

(21) Anmeldenummer: 08001291.7

(22) Anmeldetag: 24.01.2008

(84) Benannte Vertragsstaaten:
AT BE BG CH CY CZ DE DK EE ES FI FR GB GR HR HU IE IS IT LI LT LU LV MC MT NL NO PL PT RO SE SI SK TR
Benannte Erstreckungsstaaten:
AL BA MK RS

(30) Priorität: 02.02.2007  DE 102007005283

(71) Anmelder: **Cognis IP Management GmbH**
**40589 Düsseldorf (DE)**

(72) Erfinder:
• **Huesken, Hendrik**
  **45721 Haltern am See (DE)**
• **Birnbrich, Paul**
  **42719 Solingen (DE)**
• **Höfer, Rainer**
  **40477 Düsseldorf (DE)**

(54) **Lösungsmittel für Phasentransferkatalysierte Reaktionen**

(57)    Organischen Substanzen mit einem log P - Wert von mehr als 2,6 (wobei der log P-Wert experimentell gemäß der "OECD Guideline No. 107 for the testing of chemicals" vom 27.07.95 bestimmt wird) eignen sich als Lösungsmittel für Reaktionen, die durch Phasentransfer-Katalysatoren beschleunigt werden.

EP 1 953 127 A2

**Beschreibung**

**Gebiet der Erfindung**

Die Erfindung betrifft die Verwendung von organischen Substanzen mit einem log P - Wert von mehr als 2,6 als Lösungsmittel für Reaktionen, die durch Phasentransfer-Katalysatoren beschleunigt werden. Die Erfindung betrifft darüber hinaus ein Verfahren zur Herstellung von organischen Substanzen, wobei man die Reaktanden in einem System, das mindestens zwei Phasen enthält, in Gegenwart eines Phasentransfer-Katalysators miteinander umsetzt, wobei es sich bei einer der Phasen um ein organisches Lösungsmittel handelt, das einem log P - Wert von mehr als 2,6 aufweist.

**Stand der Technik**

Unter "Phasentransfer-Katalyse" (oft als PTC abgekürzt) versteht man eine in der organischen Synthese wichtige Methode, bei der in der Regel ein anionisches Reagens aus der wässrigen oder festen Phase in eine organische Phase überführt wird. Das transferierte Reagenz besitzt dann, aufgrund der geringeren Hydratisierung, der erhöhten Konzentration und der größeren Nähe zum Reaktanden eine deutlich erhöhte Reaktivität für die beabsichtigte Umsetzung. Gängige Reagenzien, die transferiert werden können, sind:

a) Basen wie $OH^-$, $HCO_3^-$
b) Nucleophile wie Fluorid, Chlorid, Bromid, Iodid, $CN^-$, $RO^-$, $RCO_2^-$
c) Oxidationsmittel wie Permanganat, Perchromat
d) Reduktionsmittel wie $BH_4^-$

Die Vorteile der PTC sind vor allem höhere Ausbeuten, mildere Reaktionsbedingungen, geringere Verunreinigungen und leichtere Aufarbeitung des Reaktionsgemisches.
Eine sehr bedeutende Anwendung findet wässriges Natriumhydroxid, das unter Phasentransfer-Bedingungen Alkoholate, Amide und Hydride ersetzen kann. Nucleophile Substitutionen, Dehydrohalogenierungen, Oxidationen und Reduktionen sowie zahlreiche andere Reaktionen, auch z.B. für die Herstellung von Polymeren wichtige Reaktionen sind der PTC zugänglich. Der Mechanismus der nucleophilen Substitution mittels PTC läßt sich am Beispiel der häufig verwendeten quartären Ammonium-Kationen plausibel machen:

Organ. Phase    $[QA]^+ X^-$ + R—Y $\longrightarrow$ R—X + $[QA]^+ Y^-$

Phasen-grenze

wäss. od. feste Phase    $[QA]^+ X^-$ + $Y^-$ $\rightleftharpoons$ $X^-$ + $[QA]^+ Y^-$

$[QA]^+$ = quartäres Ammonium-Kation

Das Ammonium-Kation bildet mit dem Reagens $X^-$ ein Ionenpaar, für das sich ein Konzentrations-Gleichgewicht zwischen den beiden Phasen einstellt. $X^-$ reagiert dann schnell in der organanischen Phase mit dem Reaktanten RY unter Bildung des Produktes RX und einem neuen Ionenpaar von $Y^-$ mit dem Ammonium-Kation. Auch für dieses stellt sich ein Konzentrations-Gleichgewicht zwischen den beiden Phasen ein, wobei der Katalysator in der wässrigen oder festen Phase das abgespaltene $Y^-$ gegen ein neues Reagenz $X^-$ austauscht und damit der Katalysezyklus von neuem beginnen kann.
Typischerweise finden PTC-katalysierte Reaktionen in einem 2-Phasen-System statt, deren eine Phase Wasser oder ein Feststoff ist und die andere Phase ein wasserunlöslicher Reaktant ist. Da bei Reaktionen die unter Phasen Tranfer Katalyse-Bedingungen durchgeführt werden die Reaktionstemperatur häufig recht niedrig gewählt werden kann (typischerweise < 80°C), wird auch häufig eine Lösungsmittel zum Lösen eines wasserunlösliche Edukts benötigt, wenn dieses zum Beispiel einen Schmelzpunkt oberhalb der Reaktionstemperatur aufweist. Allgemein ergeben sich durch den Einsatz eines Lösungsmittels folgende Vorteile:

• Man ermöglicht den Einsatz von Reaktanden mit hohem Schmelpunkt
• Man ermöglicht eine Reaktionstemperatur unterhalb des Schmelzpunktes
• Man erniedrigt gegebenenfalls die Viskosität des Eduktes und/oder Produktes und damit des Gesamtsystems
• Man ermöglicht die Umsetzung von Hydrolyse-empfindlichen Stoffen in Gegenwart von Wasser.

In der Literatur sind für alle Reaktionstypen reichlich Beispiele belegt, bei denen ein Lösungsmittel in der Phasentransferkatalyse (PTC) benötigt wird. Typische Lösungsmittel sind dabei Toluol, Benzol, MIBK, MTBE, Xylol und Dichlormethan. Diese Lösungsmittel sind jedoch doch aufgrund ihrer Gefährdungspotentiale in technischen Prozessen wie auch bereits in Laborversuchen bedenklich. In der Literatur lassen sich zwar auch Beispiele im Bereich der PTC finden, die auf den Einsatz von Lösungsmittel verzichten. In zahlreichen Fällen ist jedoch der Einsatz eines Lösungsmittels zwingend erforderlich.

**Beschreibung der Erfindung**

Aufgabe der vorliegenden Erfindung war es, Lösungsmittel für Phasentransferkatalysierte Reaktionen (PTC-Reaktionen) bereitzustellen. Diese Lösungsmittel sollten eine optimale Prozeßführung von PTC-Reaktionen ermöglichen (hohe Reaktionsgeschwindigkeiten, gute Ausbeuten). Sie sollten ferner im Vergleich zu herkömmlichen Lösungsmitteln wie Toluol, Benzol, MIBK, MTBE, Xylol und Dichlormethan ein geringeres Gefährdungspotential für Mensch und Umwelt aufweisen.

Gegenstand der vorliegenden Erfindung ist die Verwendung von organischen Substanzen X mit einem log P - Wert von mehr als 2,6 als Lösungsmittel für Reaktionen, die durch Phasentransfer-Katalysatoren beschleunigt werden, wobei der log P - Wert experimentell gemäß der "OECD Guideline No. 107 for the testing of chemicals" bestimmt wird.

Der log P - Wert einer organischen Substanz X ist wie folgt definiert:

$$\log P_X = \log \left[ \left( c_{X(\text{Wasser})} \right) / \left( c_{X(\text{n-Octanol})} \right) \right]$$

wobei $c_{\text{Wasser}}$ bzw. $c_{\text{n-Octanol}}$ jeweils die Gleichgewichtskonzentration bedeutet, die sich einstellt, wenn der organische Stoff X bei 20 °C in das binäre System Wasser/n-Octanol (die gewichtsmäßig im Verhältnis 1:1 vorliegen) eingebracht wird und wobei "log" der dekadische Logarithmus ist.

Prinzipiell wird der log P - Wert eines Stoffes X bestimmt, indem man die organische Substanz X bei 20 °C in eine 1 : 1 Mischung (gewichtsmäßig) von Wasser und n-Octanol einbringt, die Gleichgewichtseinstellung abwartet und dann die Gleichgewichtskonzentrationen von X in Wasser und n-Octanol bestimmt. Dann bildet man den Quotienten der Gleichgewichtskonzentrationen und nimmt von diesem Quotienten den dekadischen Logarithmus.

Es sei ausdrücklich festgestellt, dass der log P - Wert im Rahmen der vorliegenden Erfindung experimentell gemäß der "OECD Guideline No. 107 for the testing of chemicals (vom 27.07.95)" bestimmt wird.

Vorzugsweise liegt der log P - Wert der erfindungsgemäß einzusetzenden Lösungsmittel oberhalb von 2,6 und insbesondere oberhalb von 2,8.

In einer bevorzugten Ausführungsform werden erfindungsgemäß solche Stoffe mit einem log P Wert oberhalb von 2,8 als Lösungsmittel für PTC-Reaktionen eingesetzt, die ausgewählt sind aus der Gruppe der Fettsäuredialkylamide, insbesondere der Fettsäuredimethylamide. Unter Fettsäuren sind dabei Carbonsäuren mit 8 bis 24 C-Atomen zu verstehen, die vorzugsweise gesättigt sind. Wie dem Datenteil der vorliegenden Anmeldung entnommen werden kann, sind Fettsäuredimethylamide bei PTC-Reaktionen klassischen Lösungsmitteln im Hinblick auf die Reaktionsgeschwindigkeiten nicht nur gleichwertig, sondern sogar überlegen.
Beispiele geeigneter Fettsäuredimethylamide sind Octansäuredimethylamid, Decansäuredimethylamid, Dodecansäuredimethylamid und Tetradecansäuredimethylamid. In einer anderen Ausführungsform setzt man solche Fettsäuresäuredimethylamide als Lösungsmittel ein, die sich von einer verzweigten, gesättigten Fettsäure ableiten. Man kann auch Mischungen verschiedener Fettsäuredimethylamide einsetzen.

In einer weiteren Ausführungsform werden solche Stoffe mit einem log P Wert oberhalb von 2,6 (und vorzugsweise oberhalb von 2,8) als Lösungsmittel für PTC-Reaktionen eingesetzt, die ausgewählt sind aus der Gruppe der der Carbonsäurealkylester (besonders bevorzugt sind dabei Carbonsäuremethyl-, ethyl- oder isopropylester), Glycerin, Dicarbonsäureester und Ethyllactat.

Das erfindungsgemäß einzusetzende Lösungsmittel sollte gegenüber den Reaktionsbedingungen der gewünschten PTC-Reaktion inert sein.

In einer bevorzugten Ausführungsform weist das erfindungsgemäß einzusetzende Lösungsmittel einen Schmelzpunkt auf, der unterhalb von 20 °C liegt.

**Phasentransfer-Katalysatoren**

Phasentransfer-Katalysatoren sind im Sinne der vorliegenden Erfindung organische Substanzen, die die Umsetzung der Reaktanden zu den herzustellenden Verbindungen beschleunigen. Das Reaktionssystem ist dabei mindestens 2-phasig.

Als Phasentransferkatalysatoren können praktisch alle dem Fachmann für diesen Zweck einschlägig bekannten Substanzen verwendet werden. Geeignete Beispiele für geeignete Phasentransferkatalysatoren sind die Gruppen der Tetraalkylammoniumsalze, Benzyltrialkylammoniumsalze, Tetraalkylphosphoniumsalze, Benzyltrialkylphosphoniumsalze und deren Gemische sowie Polyethylenglykole und endgruppenverschlossene Polyethylenglykole.

Des weiteren können sich auch solche Substanzen als Phasentransfer-Katalysatoren eignen, die dem Fachmann unter dem Begriff der ionischen Flüssigkeiten bekannt sind. Unter ionischen Flüssigkeiten werden dabei nach üblicher Konvention organische Salze verstanden, die einen Schmelzpunkt unterhalb von 100 °C aufweisen. Vorzugsweise wird man solche ionischen Flüssigkeiten als Phasentransfer-Katalysatoren wählen, die einen Schmelzpunkt unterhalb von 20 °C aufweisen.

Im Rahmen der vorliegenden Erfindung sind die Ammoniumsalze gegenüber den Phosphoniumsalzen bevorzugt und insbesondere eignen sich die Tetra-n-butylammonium-, Tri-n-butylmethylammonium- und Benzyltriethylammoniumsalze mit den Anionen Chlorid, Bromid und Hydrogensulfat. Ganz besonders bevorzugt als Phasentransferkatalysator ist Trioctylmethylammoniumchlorid, das kommerziell unter der Bezeichnung Aliquat 336 erhältlich ist (Hersteller: Fa. Cognis). Ein weiterer im Rahmen der vorliegenden Erfindung bevorzugter Phasentransferkatalysator ist Aliquat HTA-1 (Hersteller: Fa. Cognis).

Der Phasentransferkatalysatoren wird in katalytischen Mengen eingesetzt. Die Menge hängt dabei von der Aktivität und Stabilität des Katalysators bei den gewählten Reaktionsbedingungen ab und ist der jeweiligen Reaktion anzupassen. Die Menge der Phasentransferkatalysatoren kann dabei zwischen 0,1 und 25 Mol-% - bezogen auf die Summe der eingesetzten Reaktanden - variieren und liegt besonders bevorzugt im Bereich von 0,5 bis 5 und insbesondere von 1 bis 3 Mol-%.

Durchführung der Phasentransfer-Katalyse

Hinsichtlich der Durchführung von PTC-Reaktionen gelten an sich keine besonderen Einschränkungen. Das Reaktionssystem ist mindestens 2-phasig.

In einer Ausführungsform werden diese Reaktionen kontinuierlich durchgeführt.

In einer Ausführungsform werden diese Reaktionen diskontinuierlich (batchweise) durchgeführt.

Bei den PTC-Reaktionen liegt mehr als eine flüssige Phase vor. In einer Variante liegen drei Phasen vor. Häufig ist eine flüssige Phase eine wässrige Phase. Dabei kann diese wässrige Phase auch auf einem Feststoff als so genannte omega-Phase vorliegen.

In einer besonders bevorzugten Ausführungsform liegen zwei Phasen vor, vorzugsweise eine wässrige und eine organische Phase. Dabei können die beiden Phasen in Mengenverhältnissen im Bereich von 90 : 10 bis 10 : 90 (gewichtsmäßig) eingesetzt werden.

Nach abgeschlossener PTC-Reaktion können zur Aufarbeitung an sich alle dem Fachmann hierfür bekannten Trennungs- und Reinigungsoperationen eingesetzt werden. Sofern die hergestellte organische Substanz nicht wasserlöslich ist und in der ionischen Flüssigkeit gelöst vorliegt, kann sie aus dieser mittels destillativer Verfahren oder durch Extraktion mit überkritischem Kohlendioxid gewonnen werden.

Ein weiterer Erfindungsgegenstand ist ein Verfahren zur Herstellung von organischen Substanzen, wobei man die Reaktanden in einem System, das mindestens zwei Phasen enthält, in Gegenwart eines Phasentransfer-Katalysators miteinander umsetzt, dadurch gekennzeichnet dass es sich bei einer der Phasen um ein organisches Lösungsmittel handelt, das einem log P - Wert von mehr als 2,6 aufweist. Ansonsten gelten für das erfindungsgemäße Verfahren hinsichtlich des Lösungsmittels alle bereits oben gemachten Ausführungen.

Hinsichtlich der Art der PTC-katalysierten Reaktion gelten für das erfindungsgemäße Verfahren keinerlei Einschränkungen. Das Verfahren ist für alle gängigen PTC-Reaktionen anwendbar. Beispielhaft seien hier genant: O-Alkylierung, C-Alkylierung, N-Alkylierung, S-Alkylierung, Veresterung, Umesterung, Oxidation, Reduktion, Oxidation, Michael Addition, Aldol-Kondensation, Kondensationsreaktionen, Dehydrohalogenierungen, Hydrolysen, Epoxidationen, Carbonylierungsreaktionen, chirale Reaktionen. Ebenfalls sind Reaktionen von hydrolyseempfindlichen Stoffen möglich.

Wie bereits gesagt (siehe oben) sollte das erfindungsgemäß einzusetzende Lösungsmittel gegenüber den Reaktionsbedingungen der gewünschten PTC-Reaktion inert sein.

Vorzugsweise wählt man das erfindungsgemäß einzusetzende Lösungsmittel so, dass es einen Schmelzpunkt aufweist, der unterhalb von 20 °C liegt.

**Beispiele**

Log P - Werte wurden experimentell gemäß "OECD Guideline No 107 for the testing of chemicals" (vom 27.07.95) bestimmt.

Die gemessenen Daten können der nachfolgenden Tasbelle entnommen warden:

| Stoff/Lösemittel | Log P bestimmt nach OECD Guideline No 107 for the testing of chemicals |
|---|---|
| MTBE | 1,12 |
| MIBK | 1.40 |
| Toluol | 2,49 |
| Benzol | 2,09 |
| Decansäuredimethylamid | 2,94 |
| Decan-/Octansäuredimethylamid | 2,83 |

Abkürzungen:

MTBE =            Methyl-tert.butyl-ether; log P - Wert = 1,06
MIBK =            Methyl-isobutyl-keto; log P - Wert = 1,31
Decansäuredimethylamid =    Agnique KE 3308 (Fa. Cognis); log P - Wert = 2,94
Decan-/Octansäuredimethylamid =   Agnique KE 3658 (Fa. Cognis); log P - Wert = 2,83
Aliquat 336 =         Phasentransfer-Katalysator (Fa. Cognis)

**Beispiel 1 (zum Vergleich)**

(C-Alkylierung von Desoxybenzoin in Gegenwart von MTBE als Lösungsmittel)

In einem mit Stickstoff inertisierten Reaktor (ca. 300mL Reaktorvolumen) wurde Desoxybenzoin (18,83g, 96mmol) zusammen mit einem fünffachen molaren Überschuss an 50%iger Natronlauge (53,82g, 480mmol) und MTBE (53,82g) vorgelegt. Dazu wurde n-Decan (3,30g) (interner Standard für die GC-Auswertung) zugegeben. Unter stetigem Rühren bei einer Rührerdrehzahl von 500 Umdrehungen pro Minute wurde die Mischung auf eine Reaktionstemperatur von 45°C erwärmt. Bei 45°C wurden 5,5g Aliquat 336 (6mol% bezogen auf Desoxybenzoin) und 14,16g (=120mol% bezogen auf Desoxybenzoin) Isopropylbromid zugegeben. Damit begann die Reaktionszeit. Die gesamte Reaktionsmenge betrug 150g, was den Reaktor etwa zur Hälfte füllte. Der Umsatz wurde mittels Gaschromatographie (GC) bestimmt.
Ergebnis: Umsatz nach 90min: 28%

**Beispiel 2 (zum Vergleich)**

(C-Alkylierung von Desoxybenzoin in Gegenwart von MIBK als Lösungsmittel)

In einem mit Stickstoff inertisierten Reaktor (ca. 300mL Reaktorvolumen) wurde Desoxybenzoin (18,83g, 96mmol) zusammen mit einem fünffachen molaren Überschuss an 50%iger Natronlauge (53,82g, 480mmol) und MIBK (53,82g) vorgelegt. Dazu wurde n-Decan (3,30g) (interner Standard für die GC-Auswertung) zugegeben. Unter stetigem Rühren bei einer Rührerdrehzahl von 500 Umdrehungen pro Minute wurde die Mischung auf eine Reaktionstemperatur von 45°C erwärmt. Bei 45°C wurden 5,5g Aliquat 336 (6mol% bezogen auf Desoxybenzoin) und 14,16g (=120mol% bezogen auf Desoxybenzoin) Isopropylbromid zugegeben. Damit begann die Reaktionszeit. Die gesamte Reaktionsmenge betrug 150g, was den Reaktor etwa zur Hälfte füllte. Der Umsatz wurde mittels GC bestimmt.
Ergebnis: Umsatz nach 90min: 75%

**Beispiel 3 (erfindungsgemäß)**

(C-Alkylierung von Desoxybenzoin in Gegenwart von Decansäuredimethylamid als Lösungsmittel).

In einem mit Stickstoff inertisierten Reaktor (ca. 300mL Reaktorvolumen) wurde Desoxybenzoin (18,83g, 96mmol) zusammen mit einem fünffachen molaren Überschuss an 50%iger Natronlauge (53,82g, 480mmol) und Decansäuredimethylamid (53,82g), vorgelegt. Dazu wurde n-Decan (3,30g) (interner Standard für die GC-Auswertung) zugegeben. Unter stetigem Rühren bei einer Rührerdrehzahl von 500 Umdrehungen pro Minute wurde die Mischung auf eine Reaktionstemperatur von 45°C erwärmt. Bei 45°C wurden 5,5g Aliquat 336 (6mol% bezogen auf Desoxybenzoin) und 14,16g (=120mol% bezogen auf Desoxybenzoin) Isopropylbromid zugegeben. Damit begann die Reaktionszeit. Die gesamte Reaktionsmenge betrug 150g, was den Reaktor etwa zur Hälfte füllte. Der Umsatz wurde mittels GC bestimmt.

Ergebnis: Umsatz nach 90min: 98%

**Beispiel 4 (erfindungsgemäß)**

C-Alkylierung von Desoxybenzoin in Gegenwart von Decan-/Octansäuredimethylamid als Lösungsmittel.

In einem mit Stickstoff inertisierten Reaktor (ca. 300mL Reaktorvolumen) wurde Desoxybenzoin (18,83g, 96mmol) zusammen mit einem fünffachen molaren Überschuss an 50%iger Natronlauge (53,82g, 480mmol) und Decan-/Octansäuredimethylamid (53,82g) vorgelegt. Dazu wurde n-Decan (3,30g) (interner Standard für die GC-Auswertung) zugegeben. Unter stetigem Rühren bei einer Rührerdrehzahl von 500 Umdrehungen pro Minute wurde die Mischung auf eine Reaktionstemperatur von 45°C erwärmt. Bei 45°C wurden 5,5g Aliquat 336 (6mol% bezogen auf Desoxybenzoin) und 14,16g (=120mol% bezogen auf Desoxybenzoin) Isopropylbromid zugegeben. Damit begann die Reaktionszeit. Die gesamte Reaktionsmenge betrug 150g, was den Reaktor etwa zur Hälfte füllte. Der Umsatz wurde mittels GC bestimmt.
Ergebnis: Umsatz nach 90min: 98%

**Beispiel 5 (zum Vergleich)**

(C-Alkylierung von Desoxybenzoin in Gegenwart von MTBE als Lösungsmittel ohne einen Phasen Transfer Katalysator)

In einem mit Stickstoff inertisierten Reaktor (ca. 300mL Reaktorvolumen) wurde Desoxybenzoin (18,83g, 96mmol) zusammen mit einem fünffachen molaren Überschuss an 50%iger Natronlauge (53,82g, 480mmol) und MTBE (53,82g,) vorgelegt. Dazu wurde n-Decan (3,30g) (interner Standard für die GC-Auswertung) zugegeben. Unter stetigem Rühren bei einer Rührerdrehzahl von 500 Umdrehungen pro Minute wurde die Mischung auf eine Reaktionstemperatur von 45°C erwärmt. Bei 45°C wurden 14,16g (=120mol% bezogen auf Desoxybenzoin) Isopropylbromid zugegeben. Damit begann die Reaktionszeit. Die gesamte Reaktionsmenge betrug 150g, was den Reaktor etwa zur Hälfte füllte. Der Umsatz wurde mittels GC bestimmt.
Ergebnis: Umsatz nach 90min: 0%

Zusammenfassung der Ergebnisse der Beispiele:

| Bsp. Nr. | Lösemittel | Katalysator | Log P[1] | Umsatz [2] [%] |
|---|---|---|---|---|
| 1 | MTBE | Aliquat 336 | 1,12 | 28 |
| 2 | MIBK | Aliquat 336 | 1,40 | 75 |
| 3 | Decansäuredimethylamid | Aliquat 336 | 2,94 | 98 |
| 4 | Decan-/Octansäuredimethylamid | Aliquat 336 | 2,83 | 98 |
| 5 | MTBE | ohne | 1,12 | 0 |
| 1) log P - Wert des Lösemittels (gemessen gemäß "OECD Guideline No 107 for the testing of chemicals" vom 27.07.95) 2) Umsatz nach 90 Minuten | | | | |

**Patentansprüche**

1. Verwendung von organischen Substanzen mit einem log P - Wert (bei 20 °C) von mehr als 2,6 als Lösungsmittel für Reaktionen, die durch Phasentransfer-Katalysatoren beschleunigt werden, wobei der log P - Wert experimentell gemäß der "OECD Guideline No. 107 for the testing of chemicals" bestimmt wird..

2. Verwendung nach Anspruch 1, wobei die als Lösungsmittel eingesetzten Substanzen inert im Hinblick auf die durchzuführenden Reaktionen sind.

3. Verwendung nach Anspruch 1 oder 2, wobei man als Lösungsmittel ein organische Substanzen mit einen Schmelzpunkt unterhalb von 20 °C einsetzt.

4. Verwendung nach einem der Ansprüche 1 bis 3, wobei man als Lösungsmittel organischen Substanzen mit einem log P - Wert von mehr als 2,8 einsetzt.

5. Verwendung nach einem der Ansprüche 1 bis 4, wobei man als Lösungsmittel gesättigte Fettsäuredimethylamide einsetzt.

6. Verfahren zur Herstellung von organischen Substanzen, wobei man die Reaktanden in einem System, das mindestens zwei Phasen enthält, in Gegenwart eines Phasentransfer-Katalysators miteinander umsetzt, **dadurch gekennzeichnet dass** es sich bei einer der Phasen um ein organisches Lösungsmittel handelt, das (bei 20 °C) einen log P - Wert von mehr als 2,6 aufweist, wobei der log P - Wert experimentell gemäß der "OECD Guideline No. 107 for the testing of chemicals" bestimmt wird.

7. Verfahren nach Anspruch 6, wobei man die Reaktion in einem 2-phasigen System durchführt, wobei eine Phase Wasser und die andere Phase ein organisches Lösungsmittel mit einem log P - Wert von mehr als 2,62 ist.

8. Verfahren nach Anspruch 7, wobei man als Lösungsmittel gesättigte Fettsäuredimethylamide einsetzt.

9. Verfahren nach einem der Ansprüche 6 bis 8, wobei man den Phasentransfer-Katalysator auswählt aus der Gruppe der Tetraalkylammoniumsalze, Benzyltrialkylammoniumsalze, Tetraalkylphosphoniumsalze, Benzyltrialkylphosphoniumsalze, Polyethylenglykole und endgruppenverschlossene Polyethylenglykole.

10. Verfahren nach einem der Ansprüche 6 bis 9, wobei der Phasentransfer-Katalysator eine ionischen Flüssigkeit ist.